# EUROPEAN PATENT APPLICATION

(11) **EP 1 810 689 A1**
(43) Date of publication of application: **25.07.2007**
(21) Application number: 05793213.9
(22) Date of filing: 14.10.2005
(51) Int. Cl.: A61K 45/00, A61K 31/785, A61K 31/787, A61P 3/10

(54) **PREVENTIVE AND/OR THERAPEUTIC AGENT FOR DIABETES**

(30) Priority: 15.10.2004 JP 2004301027; 25.05.2005 JP 2005152124
(71) Applicant: Mitsubishi Pharma Corporation, Osaka-shi, Osaka 541-0046 (JP)
(72) Inventor: SUZUKI, Kazuo, c/o Zoegene Corporation, Yokohama-shi Kanagawa 2278502 (JP); SAKAI, Kaoru, c/o Mitsubishi Pharma Corporation, Tokyo 1038405 (JP); ISHII, Shinichi, c/o Mitsubishi Pharma Corporation, Tokyo 1038405 (JP); SUGIMOTO, Kanami, Mitsubishi Pharma Corporation, Tokyo 1038405 (JP); AUWERX, Johan, c/o University Louis Pasteur IGBMC, 67404 Illkirch, Strasbourg (FR); WATANABE, Mitsuhiro, c/o University Louis Pasteur, 67404 Illkirch, Strasbourg (FR)
(74) Representative: Merkle, Gebhard
(86) International application number: PCT/JP2005/018927
(87) International publication number: WO 2006/041150

(57) **Abstract**

According to the present invention, an agent for prophylactic and/or therapeutic treatment of diabetes comprising a substance inhibiting activity of farnesoid X receptor as an active ingredient can be provided.

SEQUENCE LISTING

<110> Mitsubishi Pharma Corporation
<120> Antidiabetic Agent
<130> MP5154WO
<150> JP2004-301027
<151> 2004-10-15
<150> JP2005-152124
<151> 2005-05-25
<160> 14
<170> PatentIn version 3.1
<210> 1
<211> 26
<212> DNA
<213> Artificial
<220>
<223> Inventor: Suzuki, Kazuo; Sakai, Kaoru; Ishii, Shinichi; Sugimoto, Kanami;
Auwerx, Johan; Watanabe, Mitsuhiro
<220>
<223> primer
<400> 1
agcaactaaa caacctgcca gtacta 26
<210> 2
<211> 21
<212> DNA
<213> Artificial
<220>
<223> primer
<400> 2
gtccggatat tcaaggatgc a 21
<210> 3
<211> 22
<212> DNA
<213> Artificial

<220>
<223> primer
<400> 3
caaggagtat gcgtacctga ag 22
<210> 4
<211> 21
<212> DNA
<213> Artificial
<220>
<223> primer
<400> 4
ggctccaaga cttcacacag t 21
<210> 5
<211> 19
<212> DNA
<213> Artificial
<220>
<223> primer
<400> 5
ccacagctgc tgcagaaca 19
<210> 6
<211> 18
<212> DNA
<213> Artificial
<220>
<223> primer
<400> 6
gaagggtcgc atggcaaa 18
<210> 7
<211> 25
<212> DNA
<213> Artificial
<220>
<223> primer

<400> 7
ccggatctac cttgctgctc acttt 25
<210> 8
<211> 26
<212> DNA
<213> Artificial
<220>
<223> primer
<400> 8
tagcaggtag aatccaagcg cgaaac 26
<210> 9
<211> 23
<212> DNA
<213> Artificial
<220>
<223> primer
<400> 9
aagtgtggaa ctctctggaa ctg 23
<210> 10
<211> 23
<212> DNA
<213> Artificial
<220>
<223> primer
<400> 10
gggttatctt ggttggcttt atg 23
<210> 11
<211> 23
<212> DNA
<213> Artificial
<220>
<223> primer
<400> 11
ggcccttgta aacaacaaaa tac 23

<210> 12
<211> 23
<212> DNA
<213> Artificial
<220>
<223> primer
<400> 12
ggcaacaaga gctgacagta aat 23
<210> 13
<211> 20
<212> DNA
<213> Artificial
<220>
<223> primer
<400> 13
gcacgtctcc aatcctgaat 20
<210> 14
<211> 20
<212> DNA
<213> Artificial
<220>
<223> primer
<400> 14
tgaaccaaag ttgaccacca 20

## Description

### Technical Field

The present invention relates to a novel agent for prophylactic and/or therapeutic treatment of diabetes.

### Background Art

Farnesoid X receptor (FXR) is a nuclear receptor that binds to bile acid as a ligand, and is known to negatively regulate gene expression of cholesterol 7α hydroxylase (cyp7 a) which is a rate limiting enzyme of conversion of cholesterol into bile acid (Non-patent document 1). As a regulation mechanism thereof, a pathway is known in which expression of an inhibitory nuclear receptor, i.e., small heterodimer partner (SHP), is increased by activation of FXR, and then SHP inhibits the expression of cyp7α (Non-patent documents 2 and 3).

Medicaments that change the FXR activity are considered to be effective for diseases associated with abnormally high or low cholesterol levels. Anion exchange resins, known as hypocholesterolemic agents, inhibit the FXR activity by adsorbing bile acid which is an endogenous ligand of FXR, and thereby increase the expression of cyp7α (Non-patent document 4). As a result, synthesis of bile acid from cholesterol is promoted, and thus these medicaments have been proved to be useful as hypocholesterolemic agents.

Further, FXR is also known to form a heterodimer with retinoic acid receptor (RXR) as a nuclear receptor. The RXR is known to interact with nuclear receptors such as peroxisome proliferator-activated receptor (PPAR)γ and PPARα, and it has been suggested that changes in the FXR activity may act on various diseases such as obesity, diabetes and abnormal lipid metabolism in which PPARγ, PPARα, or the like is involved (Patent document 1). However, no report has been made which suggests that liver gluconeogenesis is inhibited by inhibition of the FXR activity via PPARγ or PPARα.

It has also been reported that the expressions of liver gluconeogenic enzymes such as phosphoenolpyruvate carboxykinase (PEPCK) and glucose-6-phosphatase (G6Pase) are inhibited via SHP by the activation of FXR (Non-patent document 5, Patent document 2).

However, in contrast, no report has been made that liver gluconeogenesis is conversely inhibited by inhibition of the FXR activity.

Further, it has also been reported that energy metabolism is increased by activation of FXR (Patent document 3).

However, no report has been made that energy metabolism is conversely increased by inhibition of the FXR activity.

As for colestimide, which is an anion exchange resin known as a hypocholesterolemic agent (trade name: Cholebine, Mitsubishi Pharma Corporation), reports have so far made on hypoglycemic action after administration over a certain period of time (Non-patent document 6) and an effect on the circadian variation of blood glucose levels in hypercholesterolemia patients also suffering from type 2 diabetes (Patent document 4). However, modes of mechanisms thereof remain unrevealed.

Further, there has been no report suggesting that currently used medicaments that decrease blood sugar levels, such as insulin formulations, sulfonylurea agents, rapid-acting insulin secretagogues, biguanides, α-glucosidase inhibitors and glitazones inhibit the FXR activity.

That is, to the best knowledge of the inventors of the present invention, there has been no report suggesting that any FXR activity inhibitor inhibits liver gluconeogenesis and increases energy metabolism.
Patent document 1: International Patent Publication WO02/20463
Patent document 2: International Patent Publication WO03/59253
Patent document 3: International Patent Publication WO03/80803
Patent document 4: International Patent Publication WO03/11308
Non-patent document 1: Science, 284(5418):1362-5, 1999
Non-patent document 2: Mol. Cell, 6, 507-515, 2000
Non-patent document 3: Mol. Cell, 6, 517-526, 2000
Non-patent document 4: J. Biol. Chem., 277(45):42588-95, 2002
Non-patent document 5: J. Biol. Chem., 279(22):23158-65, 2004
Non-patent document 6: Rinsho Iyaku (Journal of Clinical Therapeutics & Medicine), Vol. 12, No. 8, p.1641, June 1996

### Disclosure of the Invention

### Object to be Achieved by the Invention

An object of the present invention is to provide a novel agent for prophylactic and/or therapeutic treatment of diabetes.

### Means for Achieving the Object

Under the circumstances as described above, the inventors of the present invention conducted various researches to achieve the foregoing object. As a result, they found that a substance inhibiting the activity of the farnesoid X receptor was effective for prophylactic and/or therapeutic treatment of diabetes, and accomplished the present invention.

The gists of the present invention are as follows:
(1) An agent for prophylactic and/or therapeutic treatment of diabetes, which comprises a substance inhibiting activity of farnesoid X receptor as an active ingredient, and inhibits liver gluconeogenesis.
(2) An agent for prophylactic and/or therapeutic treatment of diabetes, which comprises a substance inhibiting activity of farnesoid X receptor as an active ingredient, and increases energy metabolism.
(3) The agent for prophylactic and/or therapeutic treatment of diabetes according to the aforementioned 2, wherein the increase in energy metabolism is an increase in basal metabolism.
(4) The agent for prophylactic and/or therapeutic treatment of diabetes according to the aforementioned 2, wherein the increase in energy metabolism is an increase in heat production.
(5) The agent for prophylactic and/or therapeutic treatment of diabetes according to the aforementioned 1 or 2, which increases expression level of a gene for cholesterol 7 α hydroxylase.
(6) The agent for prophylactic and/or therapeutic treatment of diabetes according to the aforementioned 1 or 2, which increases expression level of a gene for the small heterodimer partner.
(7) The agent for prophylactic and/or therapeutic treatment of diabetes according to any one of the aforementioned 1 to 6, wherein the substance inhibiting the activity of the farnesoid X receptor is a pharmaceutically acceptable anion exchange resin or a farnesoid X receptor antagonist.
(8) The agent for prophylactic and/or therapeutic treatment of diabetes according to the aforementioned 7, wherein the pharmaceutically acceptable anion exchange resin has a bile acid-adsorbing ability.
(9) The agent for prophylactic and/or therapeutic treatment of diabetes according to the aforementioned 7 or 8, wherein the pharmaceutically acceptable anion exchange resin is selected from colestimide, cholestyramine resin, colestipol, sevelamer hydrochloride and colesevelam hydrochloride.
(10) The agent for prophylactic and/or therapeutic treatment of diabetes according to the aforementioned 7 or 8, wherein the pharmaceutically acceptable anion exchange resin is an anion exchange resin synthesized by a polymerization reaction of an epichlorohydrin derivative and an amine of which typical examples are imidazole derivatives.
(11) The agent for prophylactic and/or therapeutic treatment of diabetes according to any one of the aforementioned 7 to 10, wherein the pharmaceutically acceptable anion exchange resin is colestimide.

### Effect of the Invention

According to the present invention, an agent for prophylactic and/or therapeutic treatment of diabetes containing a substance inhibiting the activity of the farnesoid X receptor as an active ingredient can be provided.

### Brief Description of the Drawings

[Fig. 1] Drawing which depicts changes in body weight.
[Fig. 2] Drawing which depicts changes in feed intake.
[Fig. 3] Drawing which depicts changes in plasma glucose levels after glucose load.
[Fig. 4] Drawing which depicts expression levels of genes for cyp7α and SHP in the liver.
[Fig. 5] Drawing which depicts expression levels of genes for gluconeogenic enzymes in the liver.
[Fig. 6] Drawings which depict expression levels of genes for cyp7α and SHP in the liver.
[Fig. 7] Drawing which depicts expression levels of genes for gluconeogenic enzymes in the liver.
[Fig. 8] Drawing which depicts changes in body weight.
[Fig. 9] Drawings which depict tissue weights.
[Fig. 10] Drawing which depicts changes in plasma glucose levels after insulin load.
[Fig. 11] Drawing which depicts changes in plasma glucose levels after glucose load.
[Fig. 12] Drawing which depicts oxygen consumptions.
[Fig. 13] Drawing which depicts expression levels of genes for cyp7α and SHP in the liver.
[Fig. 14] Drawing which depicts expression levels of genes for PGC-1 a, UCP-1 and Dio2 in a brown adipose tissue.
[Fig. 15] Drawing which depicts changes in plasma glucose levels after glucose load.
[Fig. 16] Drawing which depicts expression levels of genes for cyp7α and SHP in the liver.
[Fig. 17] Drawing which depicts changes in plasma glucose levels after glucose load.
[Fig. 18] Drawing which depicts expression levels of genes for cyp7α and SHP in the liver.
[Fig. 19] Drawing which depicts changes in plasma glucose levels after glucose load.
[Fig. 20] Drawing which depicts expression levels of genes for cyp7α and SHP in the liver.

### Best Mode for Carrying out the Invention

Hereafter, the present invention will be explained in detail.

In the present invention, the farnesoid X receptor is a nuclear receptor binding to bile acid as a ligand, which positively regulates the gene expression of the inhibitory nuclear receptor, small heterodimer partner (SHP), and negatively regulates the gene expression of cholesterol 7α hydroxylase (cyp7 a) that is a rate limiting enzyme in conversion of cholesterol into bile acid (Science, 284(5418):1362-5, 1999). The FXR activity can be determined on the basis of a gene expression level of cyp7α or SHP. The expression levels of genes for cyp7α and SHP can be determined by real time quantitative PCR (TaqMan Universal PCR Master Mix (Applied Biosystems)), or the like.

In the present invention, a substance that inhibits the activity of the farnesoid X receptor means a substance that increases the expression level of the gene for cyp7α about 2- to 15-fold and/or a substance that decreases the expression level of the gene for SHP by about 20 to 70%. Preferred examples include a substance that increases the expression level of the gene for cyp7α about 4- to 10-fold and/or a substance that decreases the expression level of the gene for SHP by about 30 to 60%, and more preferred examples include a substance that increases the expression level of the gene for cyp7α about 8-fold and/or a substance that decreases the expression level of the gene for SHP by about 50%.

In the present invention, inhibition of liver gluconeogenesis means decreases in the expression levels of genes for liver gluconeogenic enzymes such as each of glucose-6-phosphatase (G6Pase) and/or phosphoenolpyruvate carboxykinase(PEPCK) by about 20 to 70%, preferably about 30 to 70%, most preferably about 40%. The expression levels of genes for G6Pase, PEPCK and the like can be determined by the aforementioned real time quantitative PCR or the like.

In the present invention, the expression "increase in energy metabolism" means increases in basal metabolism and heat production, and the "basal metabolism" means oxygen consumption per body surface area or per unit body weight.

In the present invention, the expression "basal metabolism increases" means that oxygen consumption per unit body weight increased by about 5%, preferably about 10 to 15%.

In the present invention, the expression "heat production increases" means that expressions of each of genes encoding heat production-related proteins existing in brown adipose tissue (BAT) increases 1.3- to 2-fold, preferably about 3- to 4-fold. Examples of the heat production-related proteins in the BAT of small animals include deiodinase 2 (Dio2), uncoupler protein 1 (UCP-1), PPAR gamma coactivator 1a (PGC-1a), and the like. Dio2 is an enzyme that converts thyroxine (T4), a thyroid hormone, into triiodothyronine (T3). A primary action of T3 is to increase metabolism (increases in basal metabolism level and heat production). UCP-1 plays a role of causing uncoupling ofADP-induced oxidative phosphorylation, that is, directly converting energy, that is generated by catabolism, to heat without converting it into a high-energy phosphate compound such as ATP. PGC-la is a coactivator of nuclear transcription factors and positively regulates expressions of the genes for UCP-1 and Dio2.

In the present invention, a pharmaceutically acceptable anion exchange resin means an anion exchange resin that can be administered as a medicament, and preferred examples include anion exchange resins having a bile acid-adsorbing ability. As shown in the following examples, the anion exchange resins are not particularly limited so long as they inhibit liver gluconeogenesis and increase energy metabolism when they are administered .

An example is colestimide (2-methylimidazole-epichlorohydrin copolymers) which is the most preferred example. Although colestimide has an irregular and disordered complicated three-dimensional structure, the resin is represented by the basic structure of the following formula (I), of which structure is partially represented by the following formula (II), and can be obtained by a polymerization reaction of an epichlorohydrin derivative and an amine of which typical example is an imidazole derivatives, specifically, by the preparation method described in Japanese Patent Unexamined Publication (Kokai) No. 60-209523.

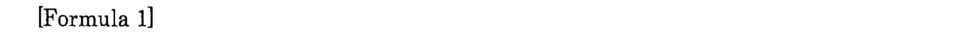

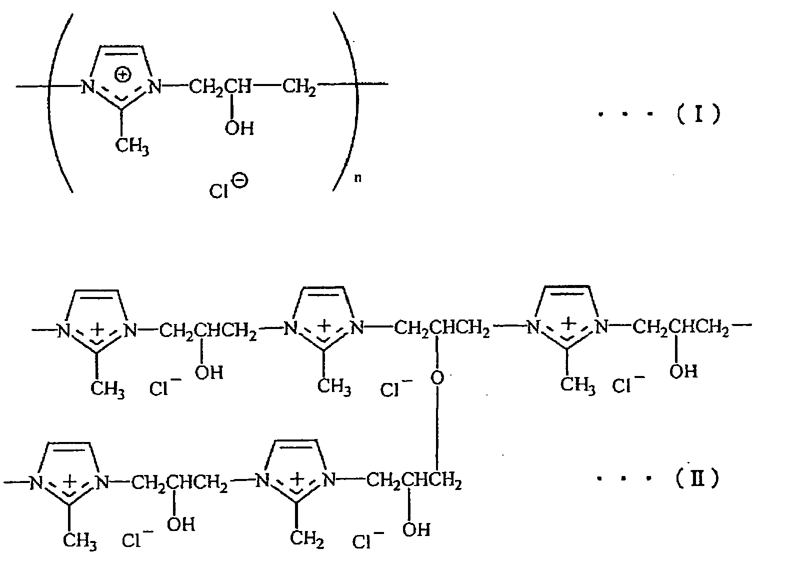

Colestimide is registered with a nonproprietary name of colestimide (chemical name: 2-methylimidazole-epichlorohydrin copolymer) in JAN. The resin is registered with a nonproprietary name of colestilan (chemical name: 2-methylimidazole polymer with 1-chloro-2,3-epoxypropane) in INN.

Other preferred examples of the anion exchange resin include the aforementioned cholestyramine resin, colestipol (N-(2-aminoethyl-N'-[2-[(2-amino-ethyl)amino]ethyl]-1,2-ethanediamine polymer added with (chloromethyl)oxylane) and the like. These resins are sold by Sigma. The cholestyramine resin is a strong basic anion exchange resin containing a styrene-divinylbenzene copolymer added with quaternary ammonium groups, and the basic structure thereof is represented by the following formula (III).

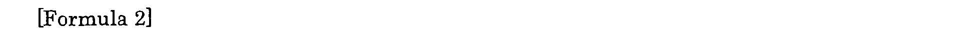

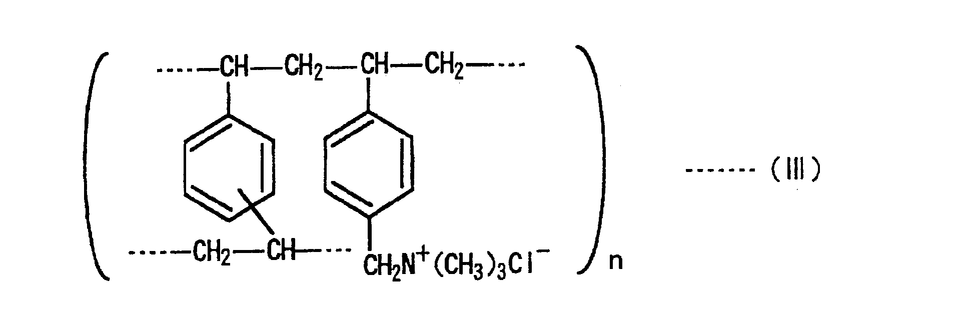

Further, the basic structure of sevelamer hydrochloride is represented by the following formula, and said resin can be prepared by the method described in U.S. Patent No. 5,496,545 or similar methods.

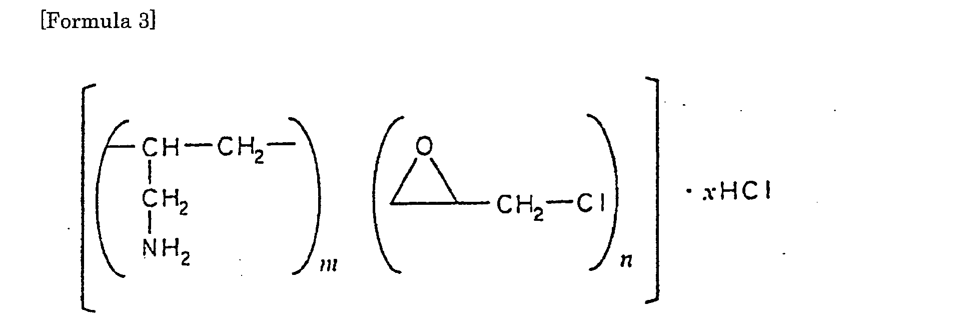

The basic structure of colesevelam hydrochloride is represented by the following formula. The resin can be prepared by the method described in U.S. Patent No. 5,607,669 or similar methods.

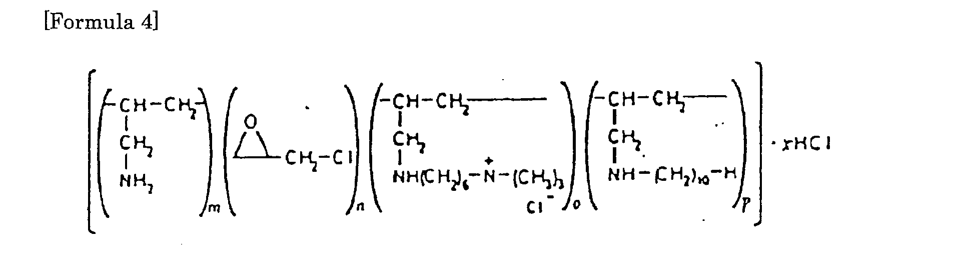

In addition, the anion exchange resins described in International Patent Unexamined Publication in Japanese (Kohyo) Nos. 9-504782, 9-500368, 10-501264, 10-501842, 11-507093, 11-512074, and 5-512332 and Japanese Patent Unexamined Publication (Kokai) Nos. 8-208750, 9-202732, 10-114661, and 11-228449 can also be used in the present invention so long as they do not depart from the gist of the present invention.

In the present invention, the farnesoid X receptor antagonist is not particularly limited so long that it inhibits liver gluconeogenesis and increases energy metabolism when administered, and may be a low molecular compound. Further, the endogenous ligands of FXR, transporters known to be involved in reabsorption of bile acid from the intestinal tract to the liver (enterohepatic circulation) and substances that inhibit bile acid-binding proteins are considered to be encompassed in the scope of the agent for prophylactic and/or therapeutic treatment of diabetes of the present invention. Specific examples of the transporters include ileal apical sodium co-dependent bile acid transporter/ileal bile acid transporter (ASBT/IBAT), Na+/taurocholate cotransporting polypeptide (NTCP), and the like, and specific examples of the bile acid-binding proteins include ileal bile acid binding protein (IBABP), and the like.

As the agent for prophylactic and/or therapeutic treatment of diabetes of the present invention, the aforementioned compounds as an active ingredient per se may be used. Pharmaceutical compositions containing the aforementioned active ingredients and commonly used additives for pharmaceutical preparations are preferably prepared and used.

Examples of such pharmaceutical compositions include tablets, capsules, subtilized granules, pills, troches, solutions and the like, and these are orally administered (including sublingual administration).

Oral pharmaceutical compositions can be prepared by conventional methods such as mixing, filling and compressing. Further, the active ingredient may be distributed in a pharmaceutical composition by using a large amount of excipient and by applying repeated mixing operations. For example, tablets or capsules used for oral administration are preferably provided as unit administration products and may contain carriers ordinarily used for pharmaceutical preparations such as binders, fillers, diluents, compressing agents, lubricants, disintegrating agents, coloring materials, flavors, and wetting agents. Tablets may be prepared as, for example, coated tablets by using a coating agent according to methods known to those skilled in the art.

Preferred examples of the excipients include cellulose, mannitol, lactose and the like. Starch, polyvinylpyrrolidone, starch derivatives such as sodium starch glycolate and the like as disintegrating agents, sodium laurylsulfate as lubricant, and the like can be used as additives for pharmaceutical preparations. Examples of pharmaceutical compositions in the form of oral liquid include pharmaceutical compositions such as aqueous or oily suspensions, solutions, emulsions, syrups and elixirs and dry pharmaceutical compositions that can be redissolved in water or a suitable medium before use.

The above solutions may be mixed with ordinary additives, for example, suspending agents such as sorbitol, syrup, methylcellulose, gelatin, hydroxyethylcellulose, carboxymethylcellulose, aluminum stearate gel and hydrogenated edible fat; emulsifiers such as lecithin, sorbitan monooleate and gum arabic; oily esters such as almond oil, rectified coconut oil and glycerin esters; non-aqueous media such as propylene glycol and ethyl alcohol (edible oil may be included); preservatives such as methyl ester, ethyl ester or propyl ester of p-hydroxybenzoic acid and sorbic acid; usual flavors or coloring materials and the like, if necessary.

Where the aforementioned oral pharmaceutical composition is in the form of, for example, a tablet, capsule, subtilized granule or the like, the composition usually contains 5 to 95% by weight, preferably 25 to 90% by weight, of the active ingredient.

Colestimide is sold by Mitsubishi Pharma Corporation with a trade name of Cholebine. Cholebine, per se, may be used according to the present invention.

A dose of the agent for prophylactic and/or therapeutic treatment of diabetes of the present invention can be suitably selected depending on the active ingredient to be used, the age, health condition and body weight of a patient, severity of diseases, type and frequency of treatment simultaneously applied, nature of desired effect, and the like. In general, as for colestimide as an example, a daily dose of 1 to 60 g for an adult in terms of the amount of the active ingredient can be administered once or several times per day.

### Examples

The present invention will be explained more specifically with reference to the following examples. However, the scope of the present invention is not limited to these examples. The colestimide used in the following examples was prepared according to the method described in Japanese Patent Unexamined Publication (Kokai) No. 60-209523.

### Example 1

### Test method

KKAy mice (male, Clea Japan, Inc., 5-week old, N = 4 or 5) were used. The control group was fed with normal diet, and the colestimide group was fed with normal diet containing 2% colestimide (UAR, Villemoison sur Orge, France).

After twelve days, oral glucose tolerance test was performed according to an ordinary method. Blood was collected before glucose load, a glucose solution was orally administered at a dose of 2 g/kg body weight, and blood sugar levels were measured 30, 60, 90, 120 minutes later.

One week later, the livers were isolated from mice, and expression levels (relative mRNA levels) of the genes for the bile acid synthetase, cyp 7 a, the inhibitory nuclear receptor, SHP, and the gluconeogenic enzymes, PEPCK and G6Pase, were measured by real time quantitative PCR.

As the primer sequences used for the real time quantitative PCR, those of SEQ ID NOS: 1 and 2 in the sequence listing were used for Cyp7α, those of SEQ ID NOS: 3 and 4 in the sequence listing for SHP, those of SEQ ID NOS: 5 and 6 in the sequence listing for PEPCK, and those of SEQ ID NOS: 7 and 8 in the sequence listing for G6Pase, respectively.

### Results

### (1) Body weight

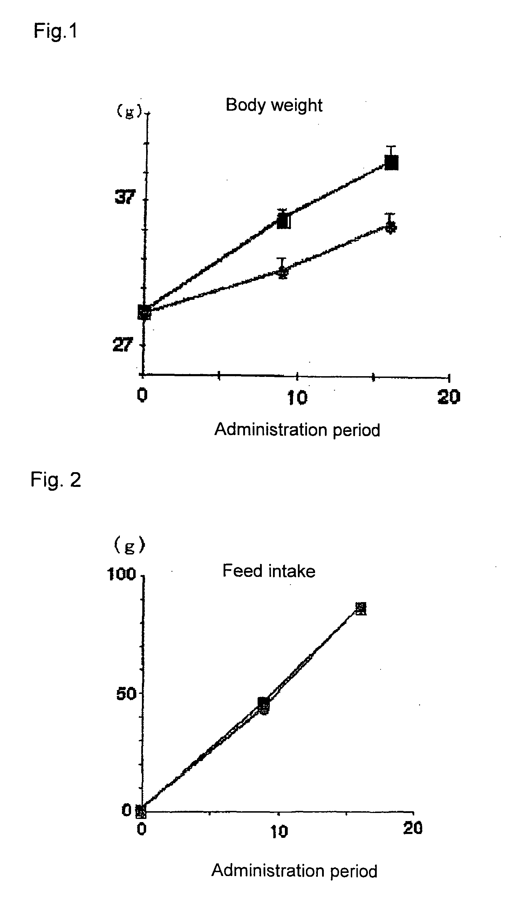

Changes in body weight in the control group (■) and the colestimide group (●) are shown in Fig. 1. As shown in Fig. 1, the body weights in the colestimide group were lower than those in the control group.

### (2) Food intake

Changes in food intake in the control group (■) and in the colestimide group (●) are shown in Fig. 2. As shown in Fig. 2, the food intakes in the colestimide group and the control group changed substantially in the same manner throughout the administration period.

### (3) Glucose tolerance test

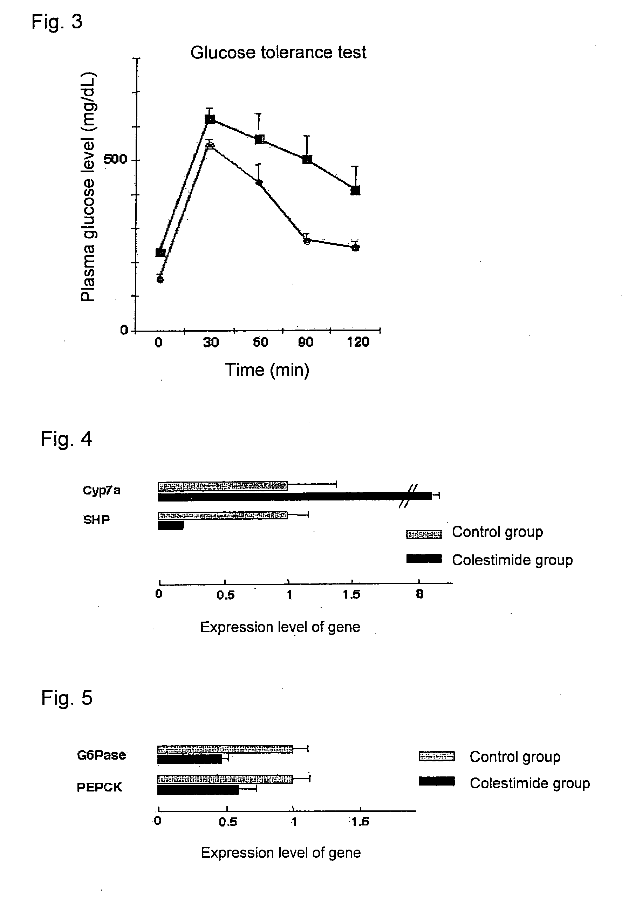

Changes in plasma glucose level (mg/ml) after the glucose load in the control group (■) and the colestimide group (●) are shown in Fig. 3. As shown in Fig. 3, the blood sugar levels decreased about 30% from those before the glucose load in the colestimide group compared with those in the control group.

Further, the blood sugar levels after the glucose load more rapidly decreased in the colestimide group compared with the control group.

### (4) Expression of genes for cyp7α and SHP in the liver

The results are shown in Fig. 4. In the colestimide group, the expression levels of the gene for cyp7α markedly increased, and were about 8 times higher than those in the control group, and the expression levels of the gene for SHP decreased 50% or more. These results suggested that the FXR activity in the liver was inhibited by colestimide, and negative regulation of bile acid synthesis was thereby eliminated.

### (5) Expression of genes for gluconeogenic enzymes in the liver

The results are shown in Fig. 5. The expression levels of genes for PEPCK and G6Pase were both suppressed in the colestimide group compared with the control group. These results suggested that gluconeogenic reactions in the liver were inhibited by colestimide.

From the results mentioned above, it was demonstrated that colestimide induced inhibition of the FXR activity in the liver and thereby inhibited liver gluconeogenesis.

### Example 2

### Test method

KKAy mice (male, Clea Japan, Inc., 5-week old, N = 6) were used. An FXR agonist, GW-4064 (J. Med. Chem., vol. 43(16), pp.2971-2974, 2000), was orally administered in a dosage of 10 mg/kg for 4 days. On the 5th day, mice were dissected to isolate the livers. The expression levels of the genes for cyp7α, SHP and PEPCK in the livers were measured by quantitative PCR. The primer sequences used for the real time quantitative PCR were the same as those mentioned in Example 1.

### Results

### (1) Expression of genes for cyp7α and SHP in the liver

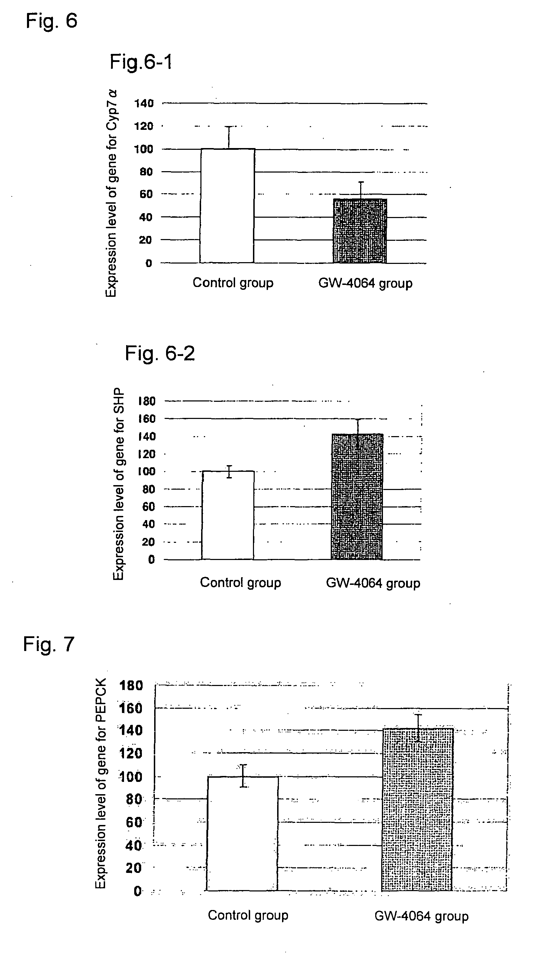

The results are shown in Fig. 6. In the GW-4064 group, expression levels of the gene for cyp7α decreased (Fig. 6-1), and expression levels of the gene for SHP increased (Fig. 6-2), compared with those in the control group. These results suggested that the FXR agonist, GW-4064, negatively regulated bile acid synthesis in the liver via increase in the FXR activity.

### (2) Expression of gene for gluconeogenic enzyme in the liver

The results are shown in Fig. 7. In the GW-4064 group, expression levels of the gene for PEPCK increased compared with that in the control group. These results suggested that the FXR agonist, GW-4064, promoted gluconeogenic reactions in the liver.

From the aforementioned results, it was demonstrated that the FXR agonist, GW-4064, induced increase in the FXR activity in the liver and thereby increased liver gluconeogenesis.

### Example 3

### Test method

C57BL6 mice (male, Charles River Laboratories, France, 1'Arbresle, France, 5-week old, N = 4 or 5) were used. Guggulipid is a medicament widely used as a drug for treatment of hyperlipemia, and a product sold by Syntrax Innovations was used in the present invention.

The control group (HFD-cont) was fed with high fat diet (35.9% fat, UAR, Villemoison sur Orge, France), the guggulipid group (HFD-guggulipid) was fed with the high fat diet containing 2.5% guggulipid, and the colestimide group (HDF-colestimide) was fed with the high fat diet containing 2% colestimide.

After 8 weeks of the administration, insulin tolerance test was performed according to an ordinary method. Blood was collected before insulin load, then insulin was intraperitoneally administered, and blood sugar levels were measured 30, 60 and 90 minutes later.

After 9 weeks of the administration, oral glucose tolerance test was performed according to an ordinary method. Blood was collected before glucose load, then a glucose solution was orally administered, and blood sugar levels were measured 30, 60, 90 and 120 minutes later.

After 12 weeks of the administration, oxygen consumption was measured by using a basal metabolism measuring apparatus (Oxymax, Columbus Instruments).

After 13 weeks of the administration, the livers, white adipose tissues surrounding the epididymis and brown adipose tissues were isolated from the mice and weighed. Further, expression levels of genes for the liver bile acid synthetase, cyp7α, the inhibitory nuclear receptor, SHP, and three proteins involved in thermogenesis in the brown adipose tissue (1. PPAR gamma coactivator 1a (PGC-1a), 2. uncoupler protein 1 (UCP-1), 3. deiodinase 2 (Dio2)) were measured by real time quantitative PCR.

As the primer sequences for the real time quantitative PCR, SEQ ID NOS: 1 and 2 in the sequence listing for Cyp7α, SEQ ID NOS: 3 and 4 in the sequence listing for SHP, SEQ ID NOS: 9 and 10 in the sequence listing for PGC-1a, SEQ ID NOS: 11 and 12 in the sequence listing for UCP-1, and SEQ ID NOS: 13 and 14 in the sequence listing for Dio2 were used.

### Results

### (1) Body weight

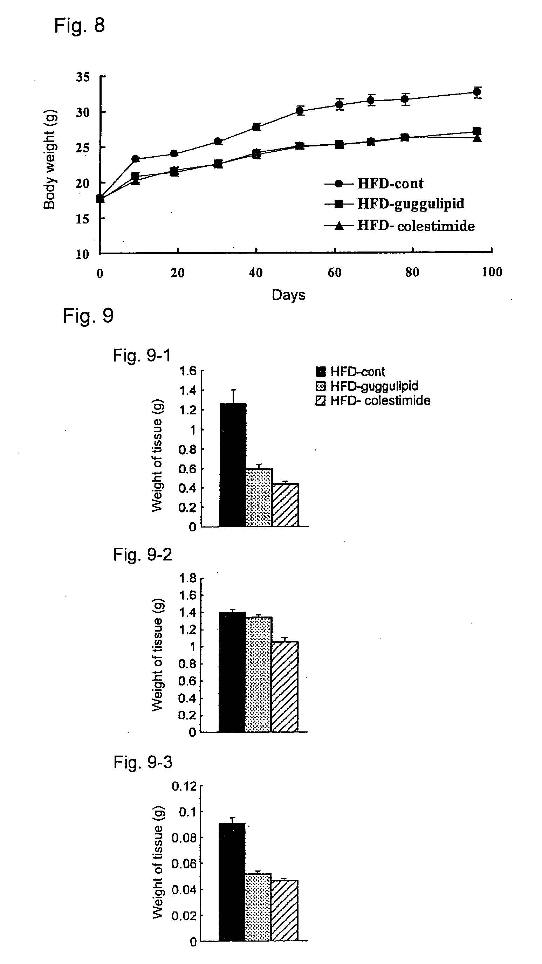

Changes in body weights in the control group (●), guggulipid group (■) and colestimide group (▲) are shown in Fig. 8. As shown in Fig. 8, body weights in the guggulipid group and the colestimide group were lower than those in the control group. (2) Organ weight
The results are shown in Fig. 9. Compared with the control group, the weights of the adipose tissues surrounding the epididymis (Fig. 9-1) and weights of the brown adipose tissues (Fig. 9-2) decreased in the guggulipid group and the colestimide group, and the weights of the livers (Fig. 9-3) also decreased in the colestimide group.

### (3) Insulin tolerance test

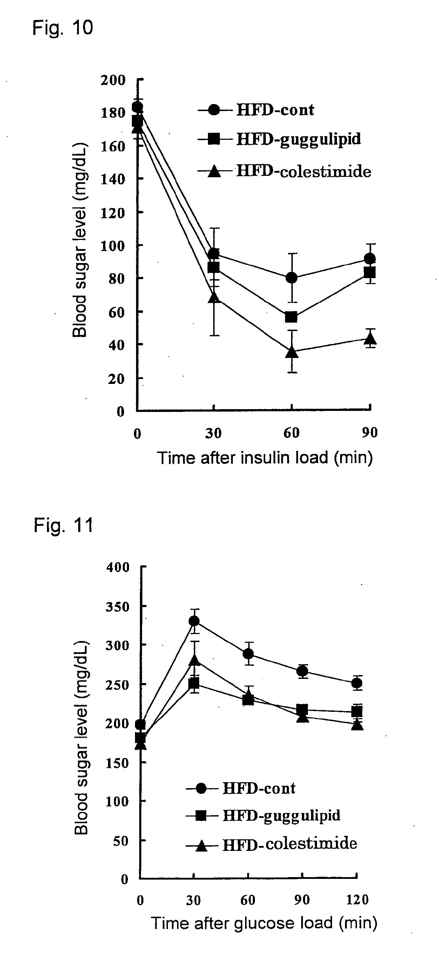

Changes in plasma glucose concentrations (mg/ml) after the insulin load in the control group (●), guggulipid group (■) and colestimide group (▲) are shown in Fig. 10. As shown in Fig. 10, the blood sugar levels after the insulin load decreased in the guggulipid group and the colestimide group compared with those in the control group.

### (4) Glucose tolerance test

Changes in plasma glucose concentrations (mg/ml) after the glucose load in the control group (●), guggulipid group (■) and colestimide group (▲) are shown in Fig. 11. As shown in Fig. 11, the blood sugar levels after the glucose load rapidly decreased in the guggulipid group and the colestimide group compared with those in the control group.

### (5) Oxygen consumption

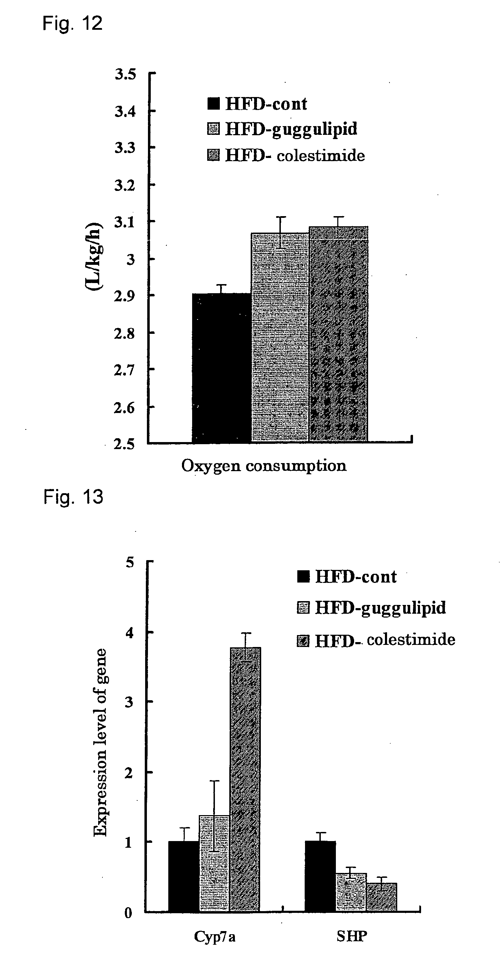

The results are shown in Fig. 12. In the guggulipid group and the colestimide group, oxygen consumptions per unit body weight increased compared with those in the control group.

These results suggested that basal metabolism was increased by guggulipid and colestimide.

### (6) Expression of genes for cyp7α and SHP in the liver

The results are shown in Fig. 13. Compared with the control group, the expression levels of the gene for cyp7α markedly increased, and the expression levels of the gene for SHP decreased about 50% in the guggulipid group and the colestimide group.

These results suggested that the FXR activity was inhibited by guggulipid and colestimide in the liver, and negative regulation of bile acid synthesis was thereby eliminated.

### (7) Expression of genes for PGC-1a, UCP-1 and Dio2 in the brown adipose tissue

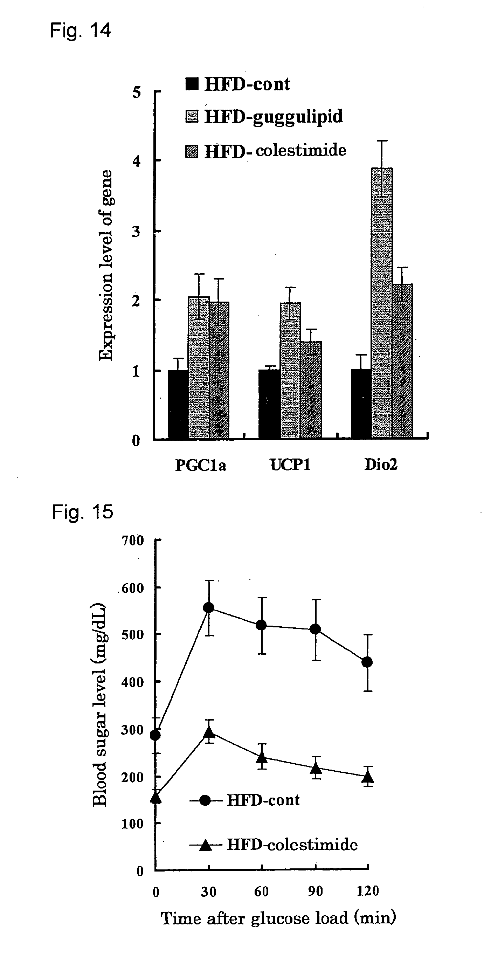

The results are shown in Fig. 14. In both the guggulipid group and the colestimide group, expression levels of all the genes increased compared with those in the control group.

These results suggested that heat production reaction in the brown adipose tissues was increased by guggulipid and colestimide.

From the results mentioned above, it was revealed that colestimide induced the inhibition of the FXR activity in the liver and thereby increased energy metabolism.

### Example 4

### Test method

KKAy mice (male, Clea Japan, Inc., N = 8) were used. The control group was fed with high fat diet (23.6% fat), and the colestimide group was fed with the high fat diet containing 2% colestimide. After 4 weeks of the administration, glucose tolerance test was performed according to an ordinary method. The mice were fasted overnight, blood was collected before glucose load, then a glucose solution was orally administered at a dose of 1 g/kg body weight, and blood sugar levels were measured 30, 60, 90 and 120 minutes later. After 6 weeks of the administration, mice were dissected to isolate the livers. The expression levels of the genes for cyp7α and SHP in the livers were measured by quantitative PCR. The primer sequences used for the real time quantitative PCR were the same as those mentioned in Example 1.

### Results

### (1) Glucose tolerance test

Changes in plasma glucose concentrations (mg/ml) after the glucose load in the control group (●) and the colestimide group (▲) are shown in Fig. 15.

As shown in Fig. 15, the blood sugar levels more rapidly decreased after the glucose load in the colestimide group compared with those in the control group.

### (2) Expression of genes for cyp7α and SHP in the liver

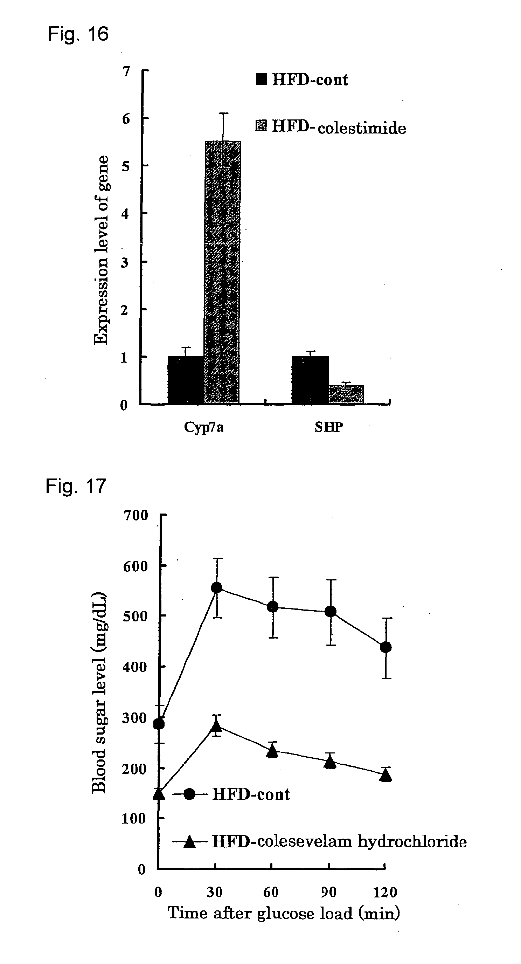

The results are shown in Fig. 16. In the colestimide group, expression levels of the gene for cyp7α increased about 5.5 times, and expression levels of the gene for SHP decreased about 60%, compared with those in the control group.

These results suggested that the FXR activity was inhibited by colestimide in the liver, and negative regulation of bile acid synthesis was thereby eliminated.

### Example 5

### Test method

KKAy mice (male , Clea Japan, Inc., N = 8) were used. The control group was fed with high fat diet (23.6% fat), and the colesevelam hydrochloride group was fed with the high fat diet containing 2% colesevelam hydrochloride. After 4 weeks of the administration, glucose tolerance test was performed according to an ordinary method. The mice were fasted overnight, blood was collected before glucose load, then a glucose solution was orally administered at a dose of 1 g/kg body weight, and blood sugar levels were measured 30, 60, 90 and 120 minutes later. After 6 weeks of the administration, mice were dissected to isolate the livers. The expression levels of the genes for cyp7α and SHP in the liver were measured by quantitative PCR. The primer sequences used for the real time quantitative PCR were the same as those mentioned in Example 1.

### Results

### (1) Glucose tolerance test

Changes in plasma glucose concentrations (mg/ml) after the glucose load in the control group (●) and the colesevelam hydrochloride group (▲) are shown in Fig. 17.

As shown in Fig. 17, the blood sugar levels more rapidly decreased after the glucose load in the colesevelam hydrochloride group compared with those in the control group.

### (2) Expression of genes for cyp7α and SHP in the liver

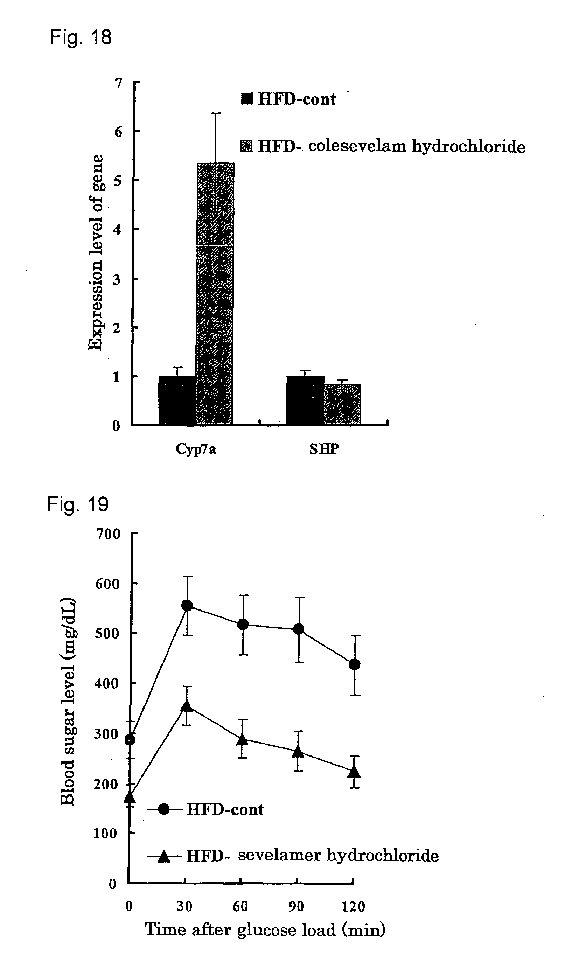

The results are shown in Fig. 18. In the colesevelam hydrochloride group, the expression levels of the gene for cyp7α increased about 5.4 times, and the expression levels of the gene for SHP decreased about 20%, compared with those in the control group.

These results suggested that the FXR activity was inhibited, and negative regulation of bile acid synthesis was eliminated by colesevelam hydrochloride in the liver.

### Example 6

### Test method

KKAy mice (male , Clea Japan, Inc., N = 8) were used. The control group was fed with high fat diet (23.6% fat), and the sevelamer hydrochloride group was fed with the high fat diet containing 2% sevelamer hydrochloride. After 4 weeks of the administration, glucose tolerance test was performed according to an ordinary method. The mice were fasted overnight, blood was collected before glucose load, then a glucose solution was orally administered at a dose of 1 g/kg body weight, and blood sugar levels were measured 30, 60, 90 and 120 minutes later. After 6 weeks of the administration, mice were dissected to isolate the livers. Expression levels of the genes for cyp7α and SHP in the livers were measured by quantitative PCR. The primer sequences used for the real time quantitative PCR were the same as those mentioned in Example 1.

### Results

### (1) Glucose tolerance test

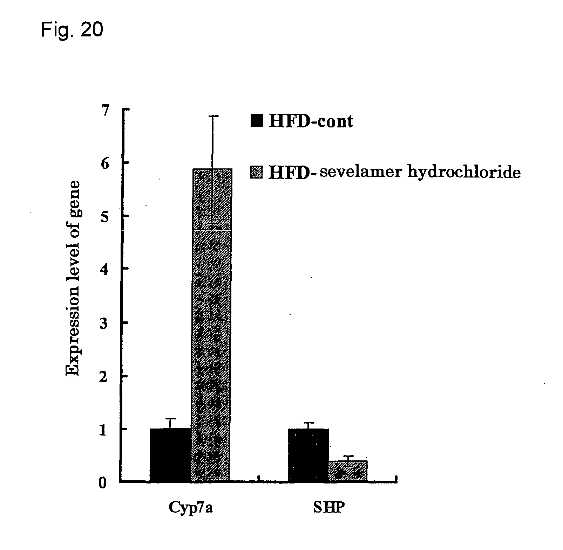

Changes in plasma glucose concentrations (mg/ml) after the glucose load in the control group (●) and the sevelamer hydrochloride group (▲) are shown in Fig. 19.

As shown in Fig. 19, the blood sugar levels more rapidly decreased after the glucose load in the sevelamer hydrochloride group compared with those in the control group.

### (2) Expression of genes for cyp7α and SHP in the liver

The results are shown in Fig. 20. In the sevelamer hydrochloride group, expression levels of the gene for cyp7α increased about 5.9 times, and the expression levels of the gene for SHP decreased about 60% compared with those in the control group.

These results suggested that the FXR activity was inhibited, and negative regulation of bile acid synthesis was eliminated by sevelamer hydrochloride in the liver.

### Industrial Applicability

According to the present invention, a novel agent for prophylactic and/or therapeutic treatment of diabetes can be provided.

This application was filed with claiming the conventional priorities based on Japanese Patent Application Nos. 2004-301027 and 2005-152124.

## Claims

1. An agent for prophylactic and/or therapeutic treatment of diabetes, which comprises a substance inhibiting activity of farnesoid X receptor as an active ingredient, and inhibits liver gluconeogenesis.

2. An agent for prophylactic and/or therapeutic treatment of diabetes, which comprises a substance inhibiting activity of farnesoid X receptor as an active ingredient, and increases energy metabolism.

3. The agent for prophylactic and/or therapeutic treatment of diabetes according to claim 2, wherein the increase in energy metabolism is an increase in basal metabolism.

4. The agent for prophylactic and/or therapeutic treatment of diabetes according to claim 2, wherein the increase in energy metabolism is an increase in heat production.

5. The agent for prophylactic and/or therapeutic treatment of diabetes according to claim 1 or 2, which increases expression level of a gene for cholesterol 7α hydroxylase.

6. The agent for prophylactic and/or therapeutic treatment of diabetes according to claim 1 or 2, which decreases an expression level of a gene for small heterodimer partner.

7. The agent for prophylactic and/or therapeutic treatment of diabetes according to any one of claims 1 to 6, wherein the substance inhibiting the activity of the farnesoid X receptor is a pharmaceutically acceptable anion exchange resin or a farnesoid X receptor antagonist.

8. The agent for prophylactic and/or therapeutic treatment of diabetes according to claim 7, wherein the pharmaceutically acceptable anion exchange resin has a bile acid-adsorbing ability.

9. The agent for prophylactic and/or therapeutic treatment of diabetes according to claim 7 or 8, wherein the pharmaceutically acceptable anion exchange resin is selected from colestimide, cholestyramine resin, colestipol, sevelamer hydrochloride, and colesevelam hydrochloride.

10. The agent for prophylactic and/or therapeutic treatment of diabetes according to claim 7 or 8, wherein the pharmaceutically acceptable anion exchange resin is an anion exchange resin synthesized by a polymerization reaction of an epichlorohydrin derivative and an amine of which typical examples are imidazole derivatives.

11. The agent for prophylactic and/or therapeutic treatment of diabetes according to any one of claims 7 to 10, wherein the pharmaceutically acceptable anion exchange resin is colestimide.
